# EUROPÄISCHE PATENTSCHRIFT

(11) **EP 0 616 511 B1**
(45) Veröffentlichungstag und Bekanntmachung des Hinweises auf die Patenterteilung: **05.03.1997**
(21) Anmeldenummer: 93900080.8
(22) Anmeldetag: 14.12.1992
(51) Int. Cl.: A61B 17/56, A61B 17/16

(54) **FÜHRUNGSVORRICHTUNG ZUM EINBRINGEN EINER SCHENKELHALSSCHRAUBE**
PILOT DEVICE FOR INSERTING A SCREW INTO THE NECK OF THE FEMUR
DISPOSITIF DE GUIDAGE PERMETTANT D'INTRODUIRE UNE VIS DANS LE COL DU FEMUR

(30) Priorität: 13.12.1991 DE 4141153
(43) Veröffentlichungstag der Anmeldung: 28.09.1994
(73) Patentinhaber: Pennig, Dietmar, Dr. med., 50935 Köln (DE)
(72) Erfinder: Pennig, Dietmar, Dr. med., 50935 Köln (DE)
(74) Vertreter: Habbel, Hans-Georg, Dipl.-Ing.
(86) Internationale Anmeldenummer: DE9201043
(87) Internationale Veröffentlichungsnummer: WO9311713

(56) Entgegenhaltungen:
- EP-A- 0 059 044
- EP-A- 0 095 296
- EP-A- 0 514 662
- CH-A- 248 631
- US-A- 4 911 153

## Beschreibung

Die Erfindung bezieht sich auf eine Führungsvorrichtung gemäß dem Oberbegriff des Hauptanspruches.

Eine gattungsbildende Führungsvorrichtung ist aus der US-PS 47 33 654 bekannt und besteht aus einer, an einem Zusatzteil zu einem Marknagel anzuschließenden Bohrschablone, die vollkommen starr ausgebildet ist, so daß weder Drehbewegungen noch Schwenkbewegungen möglich sind. Hierdurch ist die Ausrichtung der herzustellenden Bohrungen ein für alle Male festgelegt, und Anpassungen an unterschiedliche Bedingungen des einzelnen Menschen sind nicht möglich.

Der Erfindung liegt die Aufgabe zugrunde, die gattungsbildende Führungsvorrichtung so zu verbessern, daß trotz eingebrachtem Marknagel bei gebrochenem Oberschenkel eine Festlegung eines gebrochenen Oberschenkelhalses möglich wird, ohne daß der Marknagel von den der Verschraubung des Oberschenkelhalses dienenden Schrauben oder Nägel oder Knochenpins durchquert werden muß, wobei weiterhin Ausrichtungen der herzustellenden Bohrungen in Anpassung an den individuellen Einsatzfall möglich sind.

Diese der Erfindung zugrundeliegende Aufgabe wird durch die Lehre des Hauptanspruches gelöst.

Mit anderen Worten ausgedrückt wird vorgeschlagen, daß an einen üblichen Marknagel oder Verriegelungsnagel, der an seinem proximalen Ende mit einer Anschlußvorrichtung ausgerüstet ist, der Anschluß einer Führungs- oder Zielvorrichtung vorgesehen ist, die erfindungsgemäß die Bohrer so führt, daß die Bohrer beiderseits des eingesetzten Marknagels in den Oberschenkelhals bzw. den Oberschenkelkopf führen, so daß problemlos trotz eingebrachtem Marknagel anschließend ein Einschrauben der Knochenschrauben möglich ist.

Die eigentliche Zielvorrichtung besteht dabei aus einer Bohrschablone, in der wenigstens zwei quer zur Längsachse des Marknagels nebeneinander angeordnete Bohrerlöcher vorgesehen sind, die gegenüber der der Längsachse des Marknagels entsprechenden Vertikalen im wesentlichen die gleiche Neigung aufweisen wie die Achse des Schenkelhalses gegenüber der Achse des Oberschenkelknochens, d. h. etwa 130° geneigt sind, wobei aber diese beiden Löcher mit ihren Achsen in der horizontalen Ebene, d. h. in Richtung der Ausrichtung des Schenkelhalses, koinzidieren, derart, daß die Achsen sich hinter dem Schenkelkopf treffen. Die Löcher sind dabei so weit voneinander entfernt, d. h. weisen einen quer zur Längsachse des Marknagels ausgerichteten horizontalen Abstand voneinander auf, daß die eingesetzten Bohrer und anschließend die eingesetzten Schrauben problemlos an dem eingesetzten Marknagel vorbeiführen.

Die Bohrschablone wird dabei von einem Galgen getragen, der einenendes an das proximale Ende des Marknagels angreift, während das andere Ende des Galgens höhenverstellbar, d. h. in Richtung der Längsachse des Marknagels verstellbar die Bohrschablone trägt, so daß dadurch sowohl eine winkelwie auch eine höhenmäßige Einrichtung der Bohrschablone möglich ist.

Nach Einsetzen der die Fraktur des Oberschenkelhalses durchquerenden Schrauben kann der Galgen abgenommen werden, und die dann in dem Marknagel vorhandene Gewindebohrung kann durch einen entsprechenden Stopfen oder eine Schraube verschlossen werden.

Ein Ausführungsbeispiel der Erfindung wird nachfolgend anhand der Zeichnung erläutert.

Die Zeichnung zeigt dabei in
- Fig. 1: relativ schematisch teilweise aufgeschnitten einen Oberschenkelknochen mit einem Marknagel und einer angeschlossenen Bohrschablone und in
- Fig. 2: schematisch einen Schnitt zur Verdeutlichung der Achsen der Bohrerlöcher.

In der Zeichnung ist bei 1 ein Marknagel dargestellt, der in einen Oberschenkelknochen eingesetzt ist, wobei bei B eine Fraktur des Oberschenkelknochens angedeutet ist, die durch den Marknagel ruhiggestellt werden soll. Weiterhin ist eine Fraktur B im Schenkelhals dargestellt, und nunmehr müssen trotz eingesetztem Marknagel 1 Knochenschrauben durch den Oberschenkelknochen, den Schenkelhals bis in den Oberschenkelkopf geführt werden, um damit die Fraktur des Schenkelhalses ruhigzustellen.

Das proximale Ende des Marknagels ist mit 2 bezeichnet, und hier ist ein Gewindeanschluß 8 vorgesehen, in den ein entsprechendes Gegengewinde eines Galgens 6 eingeschraubt werden kann, so daß dieser Galgen 6 dann von dem proximalen Ende 2 des Marknagels 1 getragen wird.

Im nachfolgenden wird unter dem Begriff "vertikal" eine Ausrichtung verstanden, die parallel zur Längsachse des Marknagels 1 ausgerichtet ist und unter dem Begriff "horizontal" eine Ausrichtung, die quer zur Längsachse des Marknagels 1 verläuft, zum Teil auch quer zur Ausrichtung des Schenkelhalses bzw. in Richtung des Schenkelhalses.

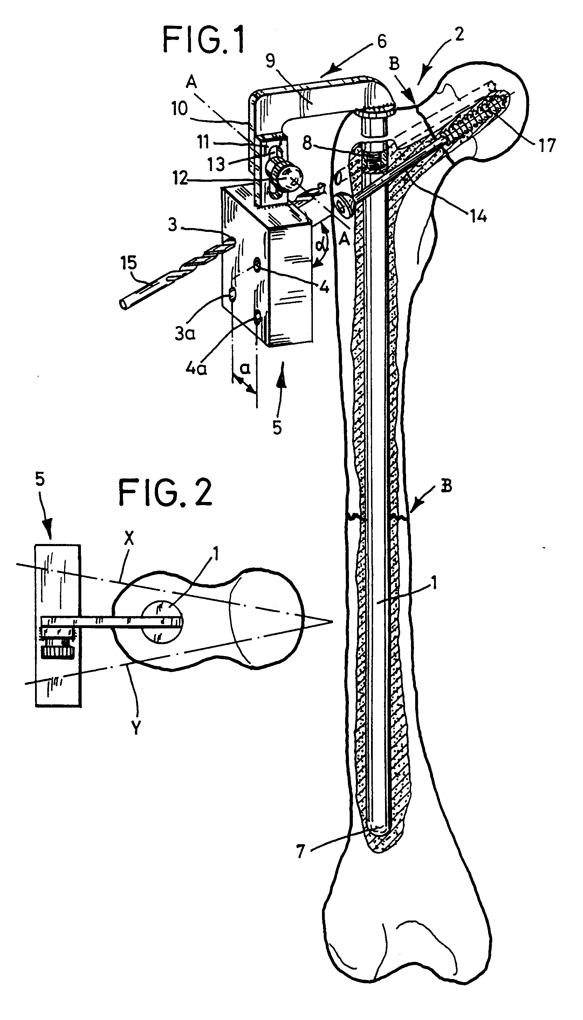

Der Galgen 6 besteht bei dem dargestellten Ausführungsbeispiel aus einem Winkelteil mit den beiden Schenkeln 9 und 10, wobei der Schenkel 10 vertikal nach unten - im wesentlichen parallel zum Marknagel 1 - gerichtet ist, während der Schenkel 9 horizontal - d. h. im wesentlichen quer zum Marknagel 1 und in Ausrichtung der Achse des Schenkelhalses - dargestellt ist und an seinem einen Ende den Gewindeanschluß 8 aufweist. An den Schenkel 10 schließt ein Halter 11 in vertikaler Richtung höhenverstellbar - d. h. in Längsachse des Schenkels 10 und des Marknagels 1 verstellbar - dadurch an, daß eine entsprechende Schraube 12 durch ein Langloch 13 zum Schenkel 10 führt, und mit dieser Schraube 12 kann der Halter 11 festgelegt werden, wobei die Schraube gleichzeitig die Gelenkachse für die horizontale Achse A - A bildet. Der Halter 11 selbst trägt eine Bohrschablone 5, die Bohrerlöcher 3 und 4 bzw. 3a und 4a aufweist, wobei diese Bohrerlöcher 3 und 4 bzw. 3a und 4a einen horizontalen Abstand a voneinander aufweisen und gegenüber der in Richtung des Schenkelhalses ausgerichteten Horizontalen in vertikaler Richtung geneigt angeordnet sind. Der Neigungswinkel α beträgt vorzugsweise 130°. Außer dieser vertikalen Neigung koinzidieren die beiden Bohrerlöcher 3 und 4 bzw. 3a und 4a in der horizontalen Ebene derart, wie dies in Fig. 2 dargestellt ist, d. h. die Achsen 5x und y treffen sich hinter dem Oberschenkelkopf, wobei aber die beiden Bohrerlöcher 3 und 4 bzw. 3a und 4a einen solchen Querabstand a voneinander aufweisen, daß die Schrauben problemlos an dem eingesetzten Marknagel 1 vorbeiführen.

Als Ausführungsbeispiel ist in der Zeichnung eine Schenkelhalsschraube 14 mit Gewinde 17 dargestellt, wobei mit 15 ein Bohrer bezeichnet ist, der die für die Aufnahme der Schraube erforderliche Bohrung vorgebohrt hat.

## Patentansprüche

1. Führungsvorrichtung für einen Bohrer zur Hersteilung der im wesentlichen quer zu einem im Oberschenkel eingebrachten Marknagel verlaufenden Bohrung zum Einbringen einer Schenkelhalsschraube mit einer an das proximale Ende (2) des Marknagels (1) abnehmbar anschließbaren Bohrschablone (5) mit wenigstens zwei Bohrerlöchern (3, 4), dadurch gekennzeichnet, daß die Bohrschablone um eine quer zur Längsachse des Marknagels (1) und quer zur Ausrichtung des Schenkelhalses verlaufende horizontale Achse (A - A) schwenkbar und parallel zur Längsachse des Marknagels (1) höhenverstellbar ist, und die Bohrerlöcher einen quer zur Längsachse des Marknagels (1) und quer zur Ausrichtung des Schenkelhalses ausgerichteten horizontalen Abstand (a) voneinander aufweisen, der mindestens dem Durchmesser des Marknagels (1) entspricht.

2. Führungsvorrichtung nach Anspruch 1, dadurch gekennzeichnet, daß die Bohrschablone (5) blockförmig ausgebildet ist und die Bohrerlöcher (3, 4; 3a, 4a) eine Neigung gegenüber der quer zur Längsachse des Marknagels (1) ausgerichteten und parallel zur Ausrichtung des Schenkelhalses verlaufenden Horizontalen aufweisen, die dem Anschlußwinkel des Schenkelhalses an den Oberschenkelknochen entspricht.

3. Führungsvorrichtung nach Anspruch 1 oder 2, gekennzeichnet durch einen um die Längsachse des Marknagels (1) drehbar am proximalen Ende des Marknagels (1) anbringbaren Galgen (6), an dessen freiem Ende parallel und in Längsachse des Marknagels (1) höhenverstellbar die Bohrschablone (5) anschließt.

4. Führungsvorrichtung nach Anspruch 3, dadurch gekennzeichnet, daß die Bohrschablone (5) um eine horizontale Achse (A - A) quer zur Längsachse des Marknagels (1) und quer zur Ausrichtung des Schenkelhalses schwenkbar an dem Galgen (6) anschließt.

5. Führungsvorrichtung nach einem der vorhergehenden Ansprüche, dadurch gekennzeichnet, daß mehrere Bohrerlöcher (3, 3a und 4, 4a) in vertikaler Richtung parallel zur Längsachse des Marknagels (1) untereinander angeordnet sind.

6. Führungsvorrichtung nach einem der vorhergehenden Ansprüche 3 - 5, gekennzeichnet durch ein Gewinde an einem Ende des Galgens (6), das in einen Gewindeanschluß (8) am proximalen Ende des Marknagels (1) einschraubbar ist.

7. Führungsvorrichtung nach einem oder mehreren der vorhergehenden Ansprüche, dadurch gekennzeichnet, daß die Achsen (x und y) der Bohrerlöcher (3, 4; 3a, 4a) quer zur Längsachse des Marknagels (1) und in Richtung der Ausrichtung des Schenkelhalses derart koinzidieren, daß diese Achsen sich hinter dem Oberschenkelkopf treffen (Fig. 2).

## Claims

1. A guide device for a drill for making the hole, extending substantially perpendicularly to a medullary nail inserted in the femur, for the insertion of a femur neck screw, comprising a drilling template (5) detachably connectable to the proximal end (2) of the medullary nail (1), said drilling template (5) comprising at least two drilling holes (3, 4), characterised in that the drilling template may pivot about a horizontal axis (A - A) extending perpendicularly to the longitudinal axis of the medullary nail (1) and perpendicularly to the orientation of the femur neck and is vertically adjustable in parallel with the longitudinal axis of the medullary nail (1), and in that the drilling holes are at a horizontal distance (a) from each other corresponding at least to the diameter of the medullary nail (1), said horizontal distance being oriented perpendicularly to the longitudinal axis of the medullary nail (1) and perpendicularly to the orientation of the femur neck.

2. A guide device according to claim 1, characterised in that the drilling template (5) is in block format and the drilling holes (3, 4; 3a, 4a) are inclined with respect to the horizontal oriented perpendicularly to the longitudinal axis of the medullary nail (1) and extending in parallel with the orientation of the femur neck, said inclination corresponding to the angle of connection of the femur neck to the femur bone.

3. A guide device according to claim 1 or claim 2, characterised by a crossbeam which may be attached to the proximal end of the medullary nail (1) so as to be rotatable about the longitudinal axis of the medullary nail (1) and to whose free end there is connected in parallel the drilling template (5), which is height adjustable along the longitudinal axis of the medullary nail (1).

4. A guide device according to claim 3, characterised in that the drilling template (5) is connected to the crossbeam (6) so as to be swivellable about a horizontal axis (A - A) perpendicularly to the longitudinal axis of the medullary nail (1) and perpendicularly to the orientation of the femur neck.

5. A guide device according to any one of the preceding claims, characterised in that a plurality of drilling holes (3, 3a and 4, 4a) are arranged one below the other in the vertical direction and in parallel with the longitudinal axis of the medullary nail (1).

6. A guide device according to any one of preceding claims 3 to 5, characterised by a thread at one end of the crossbeam (6), which may be screwed into a threaded connection (8) at the proximal end of the medullary nail (1).

7. A guide device according to any one of the preceding claims, characterised in that the axes (x and y) of the drilling holes (3, 4; 3a, 4a) perpendicular to the longitudinal axis of the medullary nail (1) and in the direction of orientation of the femur neck coincide in such a way that these axes meet behind the head of the femur (Fig. 2).

## Revendications

1. Dispositif de guidage pour un foret pour la préparation du perçage sensiblement transversal à un clou d'ostéosynthèse intramédullaire introduit dans la cuisse permettant d'introduire une vis dans le col du fémur, comprenant un gabarit de perçage (5) amovible pouvant être raccordé à l'extrémité (2) proximale du clou intramédullaire (1) et présentant au moins deux trous de forets (3, 4), caractérisé en ce que le gabarit de perçage peut pivoter autour d'un axe (A-A) horizontal et transversal à l'axe longitudinal du clou intramédullaire (1) et à l'orientation du col du fémur et est réglable en hauteur parallèlement à l'axe longitudinal du clou intramédullaire, et en ce que les trous de forets présentent entre eux un espacement (a) horizontal orienté transversalement à l'axe longitudinal du clou intramédullaire (1) et transversalement à l'orientation du col du fémur, espacement qui correspond au moins au diamètre du clou intramédullaire (1).

2. Dispositif de guidage selon la revendication 1, caractérisé en ce que le gabarit de perçage (5) est constitué sous la forme d'un bloc et les trous de forets (3, 4 ; 3a, 4a) présentent une inclinaison par rapport à l'horizontale orientée transversalement à l'axe longitudinal du clou intramédullaire (1) et s'étendant parallèlement à l'orientation du col du fémur, qui correspond à l'angle de raccordement du col du fémur sur le fémur.

3. Dispositif de guidage selon la revendication 1 ou 2, caractérisé par une potence (6) pouvant tourner autour de l'axe longitudinal du clou intramédullaire (1) et être fixée sur l'extrémité proximale du clou intramédullaire (1), ladite potence ayant une extrémité libre sur laquelle se raccorde le gabarit de perçage (5) parallèle et réglable en hauteur dans l'axe longitudinal du clou intramédullaire (1).

4. Dispositif de guidage selon la revendication 3, caractérisé en ce que le gabarit de perçage (5) peut pivoter autour d'un axe (A-A) horizontal transversalement à l'axe longitudinal du clou intramédullaire (1) et transversalement à l'orientation du col du fémur et se raccorde sur la potence (6).

5. Dispositif de guidage selon l'une quelconque des revendications précédentes, caractérisé en ce que plusieurs trous de forets (3, 3a et 4, 4a) sont disposés les uns à côté des autres dans le sens vertical parallèlement à l'axe longitudinal du clou intramédullaire (1).

6. Dispositif de guidage selon l'une quelconque des revendications 3-5 précédentes, caractérisé par un filetage sur une extrémité de la potence (6) qui peut être vissé dans un raccordement fileté (8) sur l'extrémité proximale du clou intramédullaire (1).

7. Dispositif de guidage selon l'une ou plusieurs des revendications précédentes, caractérisé en ce que les axes (x et y) des trous de forets (3, 4 ; 3a, 4a) concourent transversalement à l'axe longitudinal du clou intramédullaire (1) et dans le sens de l'orientation du col du fémur, de telle façon que ces axes se rencontrent derrière la tête du fémur (fig. 2).
